# DEMANDE DE BREVET EUROPEEN

(11) **EP 0 976 362 A1**
(43) Date de publication de la demande: **02.02.2000**
(21) Numéro de dépôt: 99401895.0
(22) Date de dépôt: 26.07.1999
(51) Int. Cl.: A61B 17/08, A61B 18/20

(54) **Dispositif pour l'assemblage des levres d'une plaie, et piece de maintien**

(30) Priorité: 27.07.1998 FR 9809557
(71) Demandeur: Galderma Research & Development, S.N.C., 06902 Sophia Antipolis cedex (FR)
(72) Inventeur: Mordon, Serge, 59491 Villeneuve D'Ascq (FR); Capon, Alexandre, 59810 Lesquin (FR); Sumian, Chryslain, Résidence les Bastides, 06600 Antibes (FR)
(74) Mandataire: Leszczynski, André

(57) **Abrégé**

Dispositif pour l'assemblage des lèvres d'une plaie.

Il comporte un laser (2) dont la longueur d'onde d'émission est choisie de manière à pouvoir réaliser une soudure tissulaire et assembler les lèvres (2) d'une plaie (P) et une pièce de maintien (4) apte à être assujettie au tissu (T) autour de la plaie (P) de manière à maintenir affrontées les lèvres (L) de cette dernière, au moins pendant l'exposition de la plaie audit rayonnement laser, la pièce de maintien (4) comportant au moins une région apte à être positionnée au-dessus de la plaie et suffisamment transparente à la longueur d'onde du rayonnement laser (6) pour que l'énergie de ce dernier, après traversée de ladite région, soit suffisante pour réaliser la soudure tissulaire recherchée.

## Description

La présente invention concerne la fermeture d'une plaie, notamment cutanée.

La suture de la peau par les techniques habituelles employant des fils ou agrafes est une procédure relativement longue et fastidieuse et présente des risques cicatriciels non négligeables, en particulier de formation d'une cicatrice hypertrophique.

Il existe depuis longtemps un besoin pour trouver un moyen de fermer rapidement les plaies sans apporter de corps étrangers tels que des fils ou des agrafes, tout en obtenant une cicatrice solide et de qualité au moins identique si ce n'est meilleure.

La soudure de la peau par laser a ainsi été proposée en tant qu'alternative à la suture par fils ou agrafes.

Toutefois, il s'est avéré que les plaies ainsi refermées présentaient une très faible résistance à la traction dans les premiers jours du processus de cicatrisation, de sorte qu'elles étaient susceptibles de se rouvrir.

On a tenté de remédier à cet inconvénient en réalisant l'irradiation par le laser en présence d'un colorant choisi pour augmenter l'absorption du rayonnement par le tissu comportant la plaie.

L'utilisation d'un tel colorant soulève toutefois des difficultés liées à la quantité de colorant déposée et à la reproductibilité du geste.

On a également proposé d'utiliser des colles à base de fibrine ou de protéines, notamment de sérum albumine humaine, destinées à apporter une plus grande solidité de la fermeture.

Cependant, l'addition de substances exogènes d'origine humaine ou animale pose des problèmes de tolérance et présente des risques de transmission de maladies virales ou spongiformes.

La présente invention vise à proposer un dispositif pour l'assemblage des lèvres d'une plaie, offrant une alternative à la suture d'un tissu par les techniques habituelles employant des fils ou agrafes, sans les inconvénients liés à l'apport d'une substance exogène ou d'un corps étranger.

Le dispositif selon l'invention se caractérise par le fait qu'il comporte un laser dont la longueur d'onde d'émission est choisie de manière à pouvoir réaliser une soudure tissulaire et assembler les lèvres de la plaie et une pièce de maintien apte à être assujettie au tissu autour de la plaie, de manière à maintenir affrontées les lèvres de cette dernière. au moins pendant l'exposition audit rayonnement laser, la pièce de maintien comportant au moins une région apte à être positionnée au-dessus de la plaie, et suffisamment transparente à la longueur d'onde du rayonnement laser pour que l'énergie de ce dernier, après traversée de ladite région, soit suffisante pour réaliser la soudure tissulaire recherchée.

Grâce à l'invention, on obtient une bonne fermeture de la plaie, car la pièce de maintien empêche toute désunion des lèvres pendant le processus de cicatrisation.

En particulier, le dispositif selon l'invention permet d'avoir au moment de l'irradiation par le laser des lèvres bien affrontées.

La cicatrisation se fait grâce à l'invention correctement et rapidement.

Par ailleurs, l'invention permet de réduire le temps nécessaire au chirurgien pour fermer la plaie, comparativement à la suture conventionnelle par fils.

Dans une réalisation particulière, la pièce de maintien est souple, et de préférence se présente sous la forme d'une bande adhésive.

Toujours dans une réalisation particulière, la pièce de maintien incorpore une ou plusieurs substances favorisant la cicatrisation et/ou ayant un effet antiseptique.

De préférence, ces substances sont choisies de telle sorte qu'elles n'aient pas d'activité directe sur la conversion de la lumière en chaleur, qui pourrait être nuisible au processus de cicatrisation recherché.

La pièce de maintien comporte avantageusement au moins une région dans sa zone de recouvrement de la plaie qui présente un coefficient d'absorption, à la longueur d'onde du rayonnement laser utilisé, choisi de telle sorte que la quantité d'énergie absorbée par ladite pièce de maintien soit au plus égale à 50% de l'énergie incidente et de préférence inférieure à 20%.

La longueur d'onde du rayonnement laser utilisé est choisie par exemple parmi les valeurs ou gammes de valeurs suivantes : 800-980 nm ; 1,06-1,32µm ; 0,514 µm ; 2,9 µm ; 10,6 µm ; et 0,805 µm, et de préférence parmi les valeurs suivantes : 810 nm. 980 nm ; 1,55 µm ; 532 nm.

L'invention a encore pour objet une pièce de maintien telle que définie plus haut.

L'invention a encore pour objet un procédé pour fermer une plaie, caractérisé par le fait qu'il comporte les étapes consistant à :
- affronter les lèvres de la plaie et coller sur le tissu à cheval sur la plaie une pièce de maintien de manière à couvrir la plaie et maintenir les lèvres de celles-ci affrontées,
- assembler les lèvres de la plaie au travers de la pièce de maintien par soudure tissulaire au moyen d'un rayonnement laser, la pièce de maintien étant suffisamment transparente à la longueur d'onde du rayonnement laser utilisé pour que l'énergie du rayonnement laser après traversée de la pièce de maintien soit suffisante pour réaliser la soudure tissulaire recherchée.

De préférence, la pièce de maintien est laissée en place sur la plaie après le traitement par le rayonnement laser pendant au moins deux jours, et de préférence pendant une période comprise entre deux et sept jours.

L'invention a encore pour objet l'utilisation lors d'une opération, notamment de chirurgie esthétique, d'un dispositif pour assembler les lèvres d'une plaie tel que défini plus haut.

L'invention a encore pour objet un procédé de traitement cosmétique de la peau permettant de diminuer les risques de formation d'une cicatrice disgracieuse lors de la cicatrisation d'une plaie, caractérisé par le fait qu'il comporte les étapes consistant à :
- appliquer sur la peau une pièce de maintien telle que définie plus haut,
- irradier la peau à travers cette pièce de maintien au moyen d'un laser tel que défini plus haut.

L'invention a encore pour objet une pièce de maintien destinée à être utilisée pour un traitement chirurgical par laser en étant appliquée sur un tissu autour des lèvres d'une plaie de manière à les maintenir dans une position prédéterminée au moins pendant l'exposition au rayonnement laser.

L'invention sera mieux comprise à la lecture de la description qui va suivre, d'un exemple de mise en oeuvre non limitatif, et à l'examen du dessin annexé sur lequel :
- la figure 1 représente de façon schématique un dispositif conforme à un exemple de réalisation de l'invention, et
- la figure 2 est une vue schématique illustrant l'irradiation de la plaie au travers de la pièce de maintien.

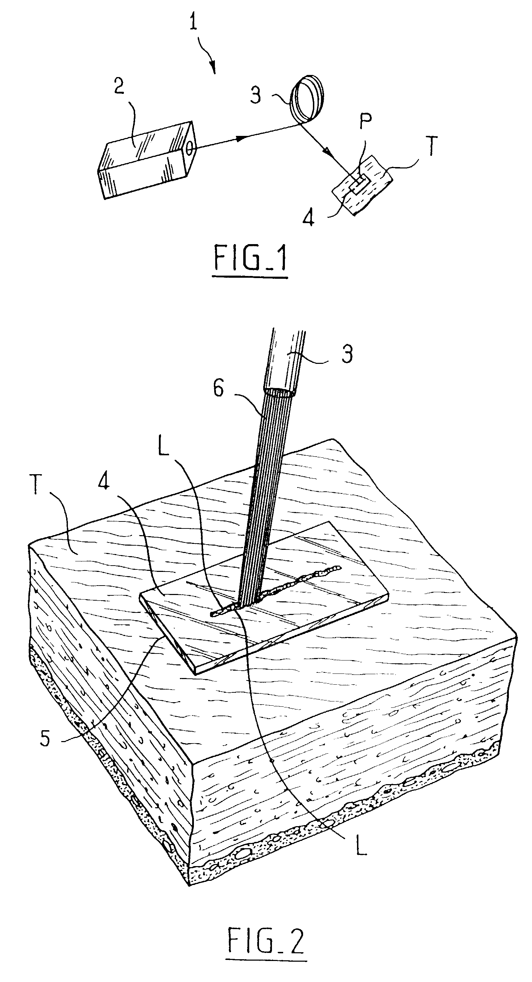

Le dispositif 1 pour l'assemblage des lèvres d'une plaie P représenté sur la figure 1 comporte d'une part un laser 2 pouvant être associé à des moyens de guidage optique comprenant une fibre optique 3 pour guider et orienter vers la plaie P le rayonnement émis par le laser et d'autre part une pièce de maintien 4 destinée à être appliquée temporairement sur le tissu T, constitué dans l'exemple décrit par la peau, pour recouvrir la plaie P.

L'utilisation d'une fibre optique 3 n'est pas indispensable et l'on peut également projeter directement à partir du laser 2 le rayonnement laser sur la plaie P ou encore diriger le rayonnement laser 2 sur un ou plusieurs miroirs orientables.

Le laser utilisé est par exemple un laser CO₂ émettant un rayonnement de longueur d'onde 10,6 µm, un laser Nd:YAG émettant dans la gamme de longueur d'onde 1,06-1,32 µm, un laser Argon émettant avec la longueur d'onde 0,514 µm, un laser Erbium émettant à 1,55 µm ou 2,9 µm ou une diode laser émettant dans la gamme 800-980 nm ou encore un laser Nd: YAC doublé émettant à 532 nm, cette liste n'étant bien entendu pas limitative.

On préfère utiliser une diode laser émettant dans la gamme 800 - 980 nm ou un laser Nd:YAG émettant dans la gamme de longueur d'onde 1,06 - 1,32 µm.

D'une manière générale, la longueur d'onde du rayonnement laser est choisie de manière à pouvoir réaliser une soudure tissulaire permettant d'assembler les lèvres L de la plaie P.

La pièce de maintien 4 est constituée dans l'exemple de réalisation décrit par une bande souple dont une face 5 est adhésive, de manière à pouvoir adhérer à la peau autour de la plaie P à refermer.

Cette bande 4 est réalisée dans l'exemple de réalisation décrit dans une matière plastique transparente à la longueur d'onde du rayonnement laser 6 émis à la sortie des moyens de guidage optique 3.

On pourrait aussi, sans sortir du cadre de la présente invention, utiliser une bande 4 réalisée dans un matériau textile par exemple.

Il faut comprendre par transparence au sens de la présente invention le fait pour la bande 4 de présenter une absorption du rayonnement 6 suffisamment faible pour que, après traversée de la bande 4, le rayonnement laser 6 ait l'énergie suffisante pour réaliser la soudure tissulaire recherchée.

La bande 4 est avantageusement constituée dans l'exemple de réalisation décrit, par un film en polyuréthanne revêtu d'une couche d'adhésif acrylique.

Le dispositif 1 s'utilise par exemple de la manière suivante.

La bande 4 est appliquée sur la peau progressivement, en la déroulant d'une extrémité à l'autre, tout en maintenant localement les lèvres L affrontées.

De préférence, la longueur de la bande 4 utilisée est supérieure à la longueur de la plaie P, comme on peut le voir sur la figure 2.

Ensuite, les lèvres L de la plaie sont assemblées par soudure tissulaire au moyen du rayonnement laser 6.

L'énergie à apporter pour réaliser cette soudure tissulaire ainsi que la localisation du point d'impact du rayonnement laser sur le tissu sont des éléments aisément déterminés par l'utilisateur en fonction du résultat à atteindre.

Le rayonnement laser 6 est, dans l'exemple décrit, appliqué continûment le long de la plaie de manière à assembler les lèvres L sur toute leur longueur.

En variante, on peut appliquer le rayonnement laser de manière discontinue en réalisant des irradiations successives, les points d'impact étant jointifs ou légèrement superposés.

Une fois les lèvres L assemblées, la bande 4 est laissée en place au moins deux jours de manière à empêcher toute désunion des lèvres L sous l'effet d'une traction exercée sur la peau.

De préférence, la bande 4 contient des substances actives permettant d'accélérer le processus de cicatrisation ainsi que, le cas échéant, des substances antiseptiques telles que de l'iodine.

On préfère utiliser pour réaliser la bande 4 un matériau qui se laisse traverser par le rayonnement laser sans le diffuser ou le diffracter sensiblement, de manière à ne pas gêner la focalisation de ce dernier en un point voulu de la plaie.

De préférence également, on préfère utiliser une bande 4 transparente non seulement vis-à-vis du rayonnement laser utilisé, mais également vis-à-vis des radiations visibles, de manière à permettre au chirurgien de suivre parfaitement l'opération de soudure tissulaire à travers la bande 4 et de surveiller ensuite le bon déroulement du processus de cicatrisation.

Des essais satisfaisants ont été réalisés en utilisant une diode laser émettant à 810 nm et des pièces de maintien constituées par des pansements adhésifs commercialisés sous les dénominations TEGADERM, OPSITE et VISULIN, qui laissent passer respectivement 88 % 96 % et 94% du rayonnement laser.

Bien entendu, l'invention n'est pas limitée à l'exemple de réalisation qui vient d'être décrit.

On peut notamment utiliser des pièces de maintien réalisées avec des formes diverses.

Ainsi, dans une variante de réalisation non représentée, on utilise une pièce de maintien présentant des ajours séparés par des ponts de matière destinés à assurer le maintien des lèvres pendant l'opération de soudure tissulaire et ultérieurement.

Cette soudure tissulaire s'effectue alors par irradiation laser au travers desdits ajours.

Dans cette variante de réalisation, la pièce de maintien peut donc être réalisée en dehors desdits ajours dans un matériau non transparent au rayonnement laser utilisé, si l'on accepte que la soudure tissulaire soit discontinue du lait de la présence desdits ponts de matière entre les ajours.

Finalement, l'invention permet de réduire le risque d'infection, la pièce de maintien pouvant assurer une protection bactérienne supplémentaire, et d'obtenir de très bons résultats sur le plan esthétique.

De plus, l'invention permet de diminuer considérablement la durée du geste opératoire.

Le temps de fermeture de la plaie peut être réduit d'un facteur trois à quatre.

A titre d'exemple, lors des essais réalisés, la durée de fermeture de la plaie a été réduite à une minute et demi au lieu de six minutes avec la suture conventionnelle par fils.

L'invention permet également d'obtenir de bons résultats en termes de vitesse de cicatrisation.

## Revendications

1. Dispositif pour l'assemblage des lèvres d'une plaie, caractérisé par le fait qu'il comporte un laser (2) dont la longueur d'onde d'émission est choisie de manière à pouvoir réaliser une soudure tissulaire et assembler les lèvres (2) d'une plaie (P) et une pièce de maintien (4) apte à être assujettie au tissu (T) autour de la plaie (P) de manière à maintenir affrontées les lèvres (L) de cette dernière, au moins pendant l'exposition de la plaie audit rayonnement laser, la pièce de maintien (4) comportant au moins une région apte à être positionnée au-dessus de la plaie et suffisamment transparente à la longueur d'onde du rayonnement laser (6) pour que l'énergie de ce dernier, après traversée de ladite région, soit suffisante pour réaliser la soudure tissulaire recherchée.

2. Dispositif selon la revendication 1, caractérisé par le fait que ladite pièce de maintien (4) est souple.

3. Dispositif selon la revendication précédente, caractérisé par le fait que ladite pièce de maintien (4) se présente sous la forme d'une bande adhésive.

4. Dispositif selon l'une quelconque des revendications précédentes, caractérisé par le fait que la pièce de maintien est constituée par un film en matière plastique, de préférence du polyuréthanne, revêtu d'une couche d'adhésif, de préférence un adhésif acrylique.

5. Dispositif selon l'une quelconque des revendications précédentes, caractérisé par le fait que ladite pièce de maintien (4) incorpore une ou plusieurs substances favorisant la cicatrisation et/ou ayant un effet antiseptique.

6. Dispositif selon l'une quelconque des revendications précédentes, caractérisé par le fait que la pièce de maintien comporte au moins une région dans sa zone de recouvrement de la plaie qui présente un coefficient d'absorption à la longueur d'onde du rayonnement laser utilisé choisi de telle sorte que la quantité d'énergie absorbée par ladite pièce de maintien soit au plus égale à 50% de l'énergie incidente et de préférence inférieure à 20%.

7. Dispositif selon l'une quelconque des revendications précédentes, caractérisé par le fait que la longueur d'onde du rayonnement laser utilisé est choisie parmi les valeurs ou gammes de valeurs suivantes : 800-980 nm ; 10,6 µm ; 1,06-1,32 µm ; 0,514 µm et 2,9 µm, et de préférence parmi les valeurs suivantes : 810 nm ; 980 nm ; 1,55µm ; 532 nm,

8. Pièce de maintien (4) telle que définie dans l'une quelconque des revendications précédentes.

9. Pièce de maintien (4) telle que définie dans l'une quelconque des revendications 1 à 7, destinée à être utilisée dans un traitement par laser, en étant appliquée sur un tissu autour des lèvres d'une plaie de manière à les maintenir dans une position prédéterminée au moins pendant l'exposition au rayonnement laser.

10. Procédé de traitement cosmétique de la peau permettant de diminuer les risques de formation d'une cicatrice disgracieuse lors de la cicatrisation d'une plaie, caractérisé par le fait qu'il comprend les étapes consistant à :
- appliquer sur la peau une pièce de maintien telle que définie dans l'une quelconque des revendications 1 à 7,
- irradier la peau à travers ladite pièce de maintien au moyen d'un laser tel que défini dans l'une quelconque des revendications 1 à 7.

11. Procédé selon la revendication 10, caractérisé par le fait que la pièce de maintien est laissée sur la peau pendant au moins deux jours, et de préférence pendant une période comprise entre deux et sept jours.
